# EUROPEAN PATENT SPECIFICATION

(11) **EP 0 819 124 B1**
(45) Date of publication and mention of the grant of the patent: **31.10.2001**
(21) Application number: 96911994.0
(22) Date of filing: 02.04.1996
(51) Int. Cl.: C07D 249/08, C07D 233/60, A61K 31/41

(54) **AZOLE COMPOUNDS WITH ANTIMYCOTIC ACTIVITY FOR HUMAN AND VETERINARY USE**
AZOLVERBINDUNGEN MIT ANTIMYKOTISCHER WIRKUNG FÜR MENSCHLICHE UND TIERÄRZLICHE VERWENDUNG
COMPOSES D'AZOLE PRESENTANT UNE ACTIVITE ANTIMYCOTIQUE A USAGES HUMAIN ET VETERINAIRE

(30) Priority: 07.04.1995 IT MI950707
(43) Date of publication of application: 21.01.1998
(73) Proprietor: ZAMBON GROUP S.p.A., 36100 Vicenza (IT); ISAGRO S.p.A., 20124 Milano (IT)
(72) Inventor: ALBINI, Enrico, I-27100 Pavia (IT); SCHIOPPACASSI, Giovanna, I-20017 Rho (IT)
(74) Representative: Panossian, Stefano
(86) International application number: EP9601443
(87) International publication number: WO9631490

(56) References cited:
- EP-A- 0 110 570
- EP-A- 0 234 242
- EP-A- 0 272 679
- EP-A- 0 315 946
- DE-A- 3 644 615
- CHEMICAL ABSTRACTS, vol. 123, no. 17, 23 October 1995 Columbus, Ohio, US; abstract no. 228190, YAMAMOTO SADAHIRO ET AL.: "Preparation of 1,2,4-triazole derivatives as fungicides" XP002006339 cited in the application & JP,A,07 070 087 (TAISHO PHARMA CO. LTD.) 14 March 1995

## Description

The present invention relates to compounds with antimycotic activity for human and veterinary use and, more particularly, it relates to azole compounds with antimycotic activity useful for the treatment and prophylaxis of infections caused by fungi and yeasts in human and in animals.

Among the antimycotic compounds known in the literature, the so-called azole derivatives among which there are some compounds used in therapy such as Fluconazole (The Merck Index, XI ed., No. 4054, page 645), Itraconazole (The Merck Index, XI ed., No. 5131, page 825) and Ketoconazole (The Merck Index, XI ed., No. 5181, page 835) represent a relevant portion.

As far as we know, however, none of these compounds is endowed with a significant antimycotic activity against some pathogenic opportunistic fungal strains which induce infections, sometimes fatal, in immunodepressed patients.

Azole fungicides for use as antimycotic agents in human and veterinary therapy are described, for example, in the German patent application DE 3644615 (Lentia GmbH), which discloses imidazole and triazole derivatives bearing an alkylamino side chain.

Other azole derivatives, characterised by the presence of a quaternary carbon atom, are reported, for example, in the European patent application No. 110570 (Pfizer Limited) that claims triazole compounds, having an halo substituted quaternary carbon atom directly linked to a fluorinated alkyl chain, as human and agricultural fungicides, or in the European patent application No. 234242 (Montedison S.p.A.) related to azoles, bearing a quaternary carbon atom substituted with H, CH₃, CN or F, for use in the agrarian field.

Among the azole derivatives known as antimycotic for human or veterinary use, several compounds have been described which are characterized by the presence of a tertiary alcohol function of formula 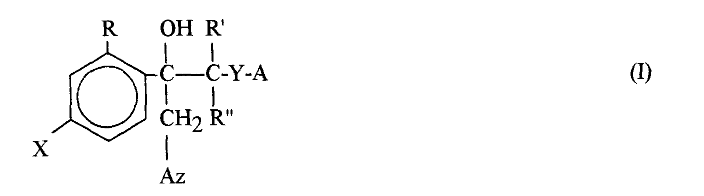 wherein Az is a triazole or imidazole group, X is preferably chlorine, fluorine or trifluoromethyl, R is preferably hydrogen, chlorine or fluorine, R' and R", the same or different, are hydrogens or alkyls, Y is S, SO, SO₂ or O and A is alkyl;
which hereinafter will be indicated with the term azole tertiary alcohols.

Among these azole tertiary alcohols we cite, for example, the compounds described in the European patent applications No. 272679 (Agrimont S.r.l.), No. 54974 (Sumitomo Chemical Company Limited), No. 61835 (Imperial Chemical Industries PLC), No. 107392 (Pfizer Limited), No. 140154 (Sumitomo Chemical Company Limited), No. 178533 (Sumitomo Pharmaceutical Company Limited), No. 435081 (SS Pharmaceutical Co. Ltd.) and No. 473387 (Sankyo Company Limited).

For some of these compounds a remarkable antimycotic activity for topic as well as for systemic use has been reported, sometimes generically. However, as far as we know, the only compound under study is a compound known as Genaconazole, (2R,3R) α-(2,4-difluorophenyl)-α-[1-(methylsulfonyl)ethyl]-1H-1,2,4-triazol-1-ethanol, described in the European patent application No. 178533.

Very recently, a class of azole tertiary alcohols (I) with antimycotic activity wherein R' and R" are fluorine atoms, Y is a simple bond and A is a difluoromethyl group has been described [Japanese patent application No. 07/70087 in the name of Taisho Pharma Co. Ltd. - Chemical Abstracts, vol. 123, No. 228190 (1995)].

We have know found that a class of azole derivatives of formula 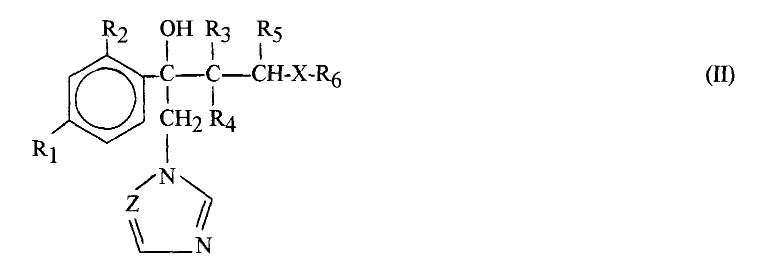 wherein
R₁ is chlorine, fluorine or trifluoromethyl;
R₂ is hydrogen, chlorine, fluorine or trifluoromethyl;
Z is CH or N;
R₃, R₄ and R₅, the same or different, are hydrogen or C₁-C₄ alkyl, provided that when one of
R₃ or R₄ is hydrogen, the other of R₃ or R₄ is different from R₅;
X is O or S;
R₆ is a C₁-C₅ polyfluoroalkyl group containing at least two fluorine atoms and optionally other halogen atoms selected among chlorine and bromine;
described in the European patent application No. 315946 (Presidenza del Consiglio dei Ministri - Ufficio del Ministero per il Coordinamento delle Iniziative per la Ricerca Scientifica e Tecnologica) as compounds for agriculture use having immunizing activity against fungal pathogenesis and phytogrowth regulating activity towards useful growings, is endowed with a potent antimycotic activity against a wide range of pathogenic fungi in human and in animals, in particular also against fungal strains resistant to antimycotics used in therapy and against opportunistic pathogenic fungal strains responsible for infections in immunodepressed patients, and is active for topical as well as for systemic route.

Therefore, object of the present inventions are compounds of formula 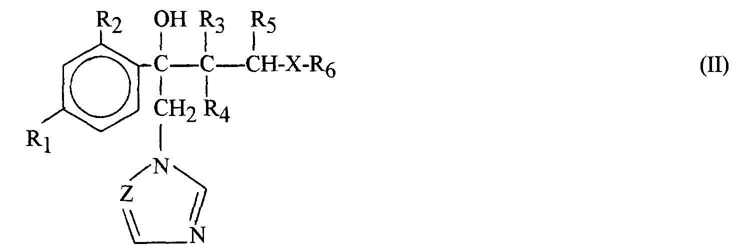 wherein
R₁ is chlorine, fluorine or trifluoromethyl;
R₂ is hydrogen, chlorine, fluorine or trifluoromethyl;
Z is CH or N;
R₃, R₄ and R₅, the same or different, are hydrogen or C₁-C₄ alkyl, provided that when one of R₃ or R₄ is hydrogen, the other of R₃ or R₄ is different from R₅;
X is O or S;
R₆ is a C₁-C₅ polyfluoroalkyl group containing at least two fluorine atoms and optionally other halogen atoms selected among chlorine and bromine;
and their salts with pharmaceutically acceptable acids;
for use as a medicament.

The compounds of formula II are endowed with a potent wide-range antimycotic activity, in particular against *Candida spp* and *Cryptococcus neoformans* strains, which are Fluconazole and Itraconazole-resistant, and against *Candida glabrata*, *Candida krusei*, *Aspergillus spp* and *Fusarium spp*, which are Itraconazole-resistant and, as the previous ones, pathogenic strains responsible for mycotic infections in immunodepressed patients, and are useful for the treatment and the prophylaxis of infections caused by fungi and yeasts in human and in animals.

The compounds of formula II contain at least a chiral center and then they can be in the form of stereoisomers.

The single stereoisomers of the compounds of formula II as well as their mixtures fall within the present invention.

With the term C₁-C₄ alkyl among the meanings of R₃, R₄ and R₅, we intend methyl, ethyl, n.propyl, isopropyl, n.butyl, isobutyl, sec.butyl and t.butyl groups, methyl and ethyl being preferred.

With the term C₁-C₅ polyfluoroalkyl group containing at least two fluorine atoms we preferably intend difluoromethyl, trifluoromethyl, 1,1,2-trifluoro-2-chloroethyl groups, 1,1-difluoroethyl, 2,2,2-trifluoroethyl, 1,1,2-trifluoroethyl, 1,1,2,3,3,3-hexafluoropropyl, 2,2,3,3-tetrafluoropropyl, 1,1,2,2-tetrafluoroethyl and their isomers; the 1,1,2,2-tetrafluoroethyl group being still more preferred.

The salts of the compounds of formula II are salts with pharmaceutically acceptable organic or inorganic acids such as chloridric, bromidric, iodidric, sulfuric, nitric, fosforic, acetic, oxalic, maleic, benzoic, benzenesulfonic, 4-methylbenzenesulfonic, fumaric, lactic, tartaric, citric and gluconic acid.

Preferred compounds of formula II are compounds wherein R₁ is chlorine or fluorine, R₂ is hydrogen, chlorine or fluorine, R₃ is methyl, R₄ and R₅, the same or different, are hydrogen, methyl or ethyl, Z is N and R₆ is a 1,1,2,2-tetrafluoroethyl group.

Still more preferred compounds of formula II are compounds wherein R₁ is chlorine or fluorine, R₂ is hydrogen, chlorine or fluorine, R₃ is methyl, R₄ and R₅, the same or different, are hydrogen, methyl or ethyl, Z is N and R₆ is a 1,1,2,2-tetrafluoroethyl group and X is O.

Specific examples of preferred compounds of formula II are the following:
1-(1H-1,2,4-triazolyl)-2-hydroxy-2-(2,4-dichlorophenyl)-3 -methyl-4-(1,1,2,2-tetrafluoroethoxy)butane
1-(1H-1,2,4-triazolyl)-2-hydroxy-2-(2,4-dichlorophenyl)-3-methyl-4-(1,1,2,2-tetrafluoroethylthio)butane
1-(1H-1,2,4-triazolyl)-2-hydroxy-2-(2,4-difluorophenyl)-3 -methyl-4-(1,1,2,2-tetrafluoroethoxy)butane
1-(1H-1,2,4-triazolyl)-2-hydroxy-2-(2,4-difluorophenyl)-3-methyl-4-(1,1,2,2-tetrafluoroethylthio)butane
1 -(1H-1,2,4-triazolyl)-2-hydroxy-2-(4-chlorophenyl)-3-methyl-4-(1,1,2,2-tetrafluoroethoxy)-butane
1-(1H-1,2,4-triazolyl)-2-hydroxy-2-(4-chlorophenyl)-3 -methyl-4-(1,1,2,2-tetrafluoroethylthio)butane
1-(1H-1,2,4-triazolyl)-2-hydroxy-2-(2,4-difluorophenyl)-3,3-dimethyl-4-(1,1,2,2-tetrafluoroethoxy)butane
1-(1H-1,2,4-triazolyl)-2-hydroxy-2-(2,4-difluorophenyl)-3,3-dimethyl-4-(1,1,2,2-tetrafluoroethylthio)butane
1-(1H-1,2,4-triazolyl)-2-hydroxy-2-(4-fluorophenyl)-3-methyl-4-(1,1,2,2-tetrafluoroethoxy)-butane
1-(1H-1,2,4-triazolyl)-2-hydroxy-2-(4-fluorophenyl)-3-methyl-4-(1,1,2,2-tetrafluoroethylthio)-butane
1-(1H-1,2,4-triazolyl)-2-hydroxy-2-(4-chlorophenyl)-3,3-dimethyl-4-(1,1,2,2-tetrafluoroethoxy)butane
1-(1H-1,2,4-triazolyl)-2-hydroxy-2-(4-chlorophenyl)-3,3-dimethyl-4-(1,1,2,2-tetrafluoroethylthio)butane
1 -(1H-1,2,4-triazolyl)-2-hydroxy-2-(4-fluorophenyl)-3,3 -dimethyl-4-(1,1,2,2-tetrafluoroethoxy)butane
1-(1H-1,2,4-triazolyl)-2-hydroxy-2-(4-fluorophenyl)-3,3-dimethyl-4-(1,1,2,2-tetrafluoroethylthio)butane
1 -(1H-1,2,4-triazolyl)-2-hydroxy-2-(2,4-dichlorophenyl)-3 -methyl-4-(1,1,2,2-tetrafluoroethoxy)hexane
1 -(1H-1,2,4-triazolyl)-2-hydroxy-2-(2,4-dichlorophenyl)-3-methyl-4-(1,1,2,2-tetrafluoroethylthio)hexane
1-(1H-1,2,4-triazolyl)-2-hydroxy-2-(2,4-difluorophenyl)-3 -methyl-4-(1,1,2,2-tetrafluoroethoxy)hexane
1 -(1H-1,2,4-triazolyl)-2-hydroxy-2-(2,4-difluorophenyl)-3 -methyl-4-(1,1,2,2-tetrafluoroethylthio)hexane
1-(1H-1,2,4-triazolyl)-2-hydroxy-2-(2,4-dichlorophenyl)-3,3-dimethyl-4-(1,1,2,2-tetrafluoroethoxy)butane
1 -(1H-1,2,4-triazolyl)-2-hydroxy-2-(2,4-dichlorophenyl)-3,3-dimethyl-4-(1,1,2,2-tetrafluoroethylthio)butane

The preparation of the compounds of formula II can be carried out according to synthetic processes described in the already cited European patent application No. 315946.

An alternative method for the preparation of the compounds of formula II wherein X is O and R₆ is a trifluoromethyl group consists in treating an alcohol intermediate of formula 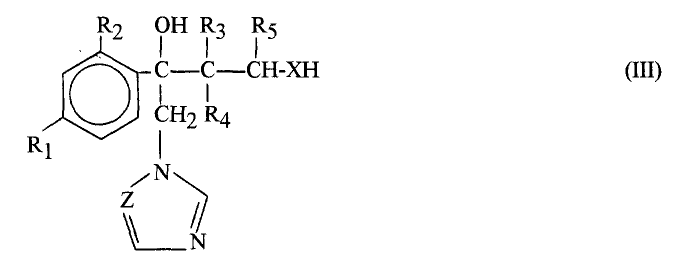 wherein R₁, R₂, R₃, R₄, R₅ and Z have the already reported meanings and X is O, with carbon disulphide and methyliodide under basic conditions and in the subsequent fluorination of the resultant methyldithiocarbonate.

The separation of the stereoisomers of formula II can be carried out with conventional techniques such as fractionated crystallization and chromatography.

The preparation of the salts of the compounds of formula II can be carried out according to conventional techniques, for example by mixing in solution equimolar amounts of the compound of formula II and of the selected acid and by separating the salt by precipitation and filtration or by evaporation of the solvent.

The compounds of formula II and their salts are antimycotic compounds useful for the treatment and the prophylaxis of infections caused by fungi and yeasts in human and animals.

In fact, the compounds of formula II , object of the present invention, are endowed with antimycotic activity against yeasts, filamentosous fungi, dermatophyti and dimorphic fungi.

The antimycotic activity has been evaluated in vitro as IC₅₀ and as MIC (Minimum Inhibiting Concentration) against numerous strains such as, for example, *Candida albicans*, *Cryptococcus neoformans*, *Trichophyton mentagrophytes*, *Aspergillus fumigatus*, *Candida parapsilosis, Candida lusitaniac*, *Candida kefyr*, *Candida tropicalis*, *Candida krusei*, *Candida glabrata*, *Aspergillus niger* and *Fusarium spp*.

It is worth underlining that the compounds of formula II, object of the present invention, resulted to be active against all the tested *Candida spp*. and *Cryptococcus neoformans* strains, comprised those Fluconazole, Itraconazole and Genaconazole-resistant.

A particularly significant antimycotic activity also resulted against *Candida glabrata* and *Fusarium spp*. strains, Itraconazole and Genaconazole-resistant, and against *Candida krusei* and *Aspergillus fumigatus*, Fluconazole and Genaconazole-resistant, all pathogenic strains responsible for infections in immunodepressed patients.

The in vivo antimycotic activity has been evaluated, by intraperitoneal and by oral route, in a *Candida* model experimentally induced in mouse, against *Candida albicans* strains sensible to Fluconazole and Itraconazole.

From the in vivo experiments, the protective dose 50% (PD₅₀) of the compounds of formula II has been also determined and it resulted to be at least comparable to that of the reference compounds.

The compounds of formula II, object of the present invention, are therefore active on wide-range deep mycosis, but in particular against opportunistic pathogens responsible for infections in immunodepressed patients, administrable by topic, oral and parenteral route and endowed with a good therapeutic index.

The compounds of formula II are then useful in human or veterinary therapy for the treatment and prophylaxis of systemic and mucosal infections caused by fungi and yeasts.

The significant pharmacological activity of the compounds of formula II which, as already underlined, also results against strains resistant to antimycotic used in therapy and against recently isolated strains responsible for infections in immunodepressed patients, is particularly surprising in that such compounds are described in the European patent application No. 315946 as compounds for agriculture use only, having immunizing activity against fungal pathogenesis and phytoregulating activity on useful growings.

The pharmacological activity of the compounds of formula II is still more surprising if it is considered that such compounds, while having some structural requirements common to the azole tertiary alcohols described in the literature, are characterized by the presence of a chain with two carbon atoms between the carbon atom bearing the hydroxy group and the oxygen or sulphur atom and by the presence of a polyfluorinated alkyl in the ether or thioether function.

These structural requirements have never been described in the literature in combination each other, as far as we know, within the field of compounds of the class of azole tertiary alcohols with antifungal activity.

In this connection, the peculiarity of the structural requirements of the compounds of formula II, object of the present invention, is extremely surprising by considering that compounds having very similar structural requirements, such as the compound identified as R1 and R2 namely 1 -(1H-1,2,4-triazolyl)-2-hydroxy-2-(2,4-difluorophenyl)-3.3-dimethyl-4-ethoxybutane and 1-(1H-1,2,4-triazolyl)-2-hydroxy-2-(2,4-difluorophenyl)-3-(2,2,3,3-tetrafluoropropoxy)-butane, are substantially inactive in comparison with the compounds of formula II.

For the human or veterinary use, the compounds of formula II can be administered in admixture with a suitable carrier depending on the kind of administration route.

The compounds of formula II or their salts can be orally administered in the form of tablets, capsules, solutions or suspensions.

For the parenteral administration, for example by intravenous, intramuscular or subcutaneous route, the compounds of formula II or their salts will be in the form of sterile aqueous solutions.

Alternatively, the compounds of formula II or their salts can be administered in the form of suppositories or pessaries.

For the topical administration the compounds of formula II or their salts will be preferably formulated as creams or powders.

For the oral or parenteral administration the daily dose of the compound of formula II will be generally from 0.1 to 50 mg/kg, preferably from 1 and 20 mg/kg, to be divided in one or more intervalled doses.

In order to better illustrate the present invention the following examples are now given.

### Example 1

The following compounds were prepared according to the procedures described in the European patent application No. 315946.

### (±)-1-(1H-1,2,4-triazolyl)-2-hydroxy-2-(2,4-dichlorophenyl)-3-methyl-4-(1,1,2,2-tetrafluoroethoxy)butane (Compound 1)

### (±)-1-(1H-1,2,4-triazolyl)-2-hydroxy-2-(2,4-dichlorophenyl)-3-methyl-4-(1,1,2,2-tetrafluoroethoxy)hexane (Compound 2)

### (±)-1-(1H-1,2,4-triazolyl)-2-hydroxy-2-(4-chlorophenyl)-3,3-dimethyl-4-(1,1,2,2-tetrafluoroethoxy)butane (Compound 3)

### (±)-1-(1H-1,2,4-triazolyl)-2-hydroxy-2-(4-chlorophenyl)-3-methyl-4-(1,1,2,2-tetrafluoroethoxy)butane (Compound 4)

### Example 2

### Preparation of (±)-1-(1H-1,2,4-triazolyl)-2-hydroxy-(2,4-difluorophenyl)-3,3-dimethyl-4-(1,1,2,2-tetrafluoroethoxy)butane (Compound 5)

Potassium hydroxide powder (5.33 mg; 9.50 mmoles) was added to a solution of (±)-3-(2,4-difluorophenyl)-2,2-dimethyl-4-(1H-1,2,4-triazolyl)-1,3-butanediol (5 g; 16.8 mmoles) and dimethylsulphoxide (8 ml) in toluene (60 ml), under stirring at -5°C.

The reaction atmosphere was substituted by tetrafluoroethylene and the mixture was kept under stirring at -5°C for 90 minutes.

After addition of water (120 ml), the organic phase was washed with 5% hydrochloric acid (80 ml) and treated with anhydrous sodium bicarbonate (6.5 g) under stirring for 30 minutes.

The liquid phase was filtered and the solvent was evaporated under reduced pressure to give an oil residue which was purified by flash chromatography (eluent hexane:ethyl acetate=6:4).

Compound 5 (5.7 g; 85% yield) was obtained as a white solid.
m.p. 81-82.5°C
¹H-NMR (CDCl₃): 8.01 (d, 1H, CH-tetr.); 7.69 (s, 1H, CH-cat.); 7.63-6.56 (m, 3H, Ar); 5.70 (tt, 1H, JHF=53.4 Hz, CHF₂); 5.33 (bs, 1H, OH); 5.29-4.41 (m, 2H, CH₂-triazole), AB system: VA=4.19, VB=3.75, JAB=9.8 Hz, CH₂O; 1.06 (d, 3H, JHF=2.4 Hz, CH₃); 0.98 (s, 3H, CH₃).

By working in a similar way the following compounds were prepared.

### (±)-1-(1H-1,2,4-triazolyl)-2-hydroxy-2-(2.4-difluorophenyl)-3-methyl-4-(1,1,2,2-tetrafluoroethoxy)butane (Compound 6) - 88% yield

m.p. 112-114°C
¹H-NMR (CDCl₃): 7.81 and 7.74 (2s, 2H, tetr.); 7.36-6.64 (m, 3H, Ar); 5.73 (tt, 1H, JHF=52.9 Hz, CHF₂); 4.99 (bs, 1H, OH); 4.95-4.53 (m, 2H, CH₂-triazole), AB portion of an ABX system: VA=4.46, VB=3.94, JAB=10.2 Hz, JAX=7.3 Hz, JBX=4.9 Hz, CH₂O; 2.63-2.45 (m, 1H, CHCH₃); 0.75 (d, 3H, CH₃CH).

### (±)-1-(1H-1,2,4-triazolyl)-2-hydroxy-2-(2,4-difluorophenyl)-3-ethyl-4-(1,1,2,2-tetrafluoroethoxy)butane nitrate (Compound 7) - 80% yield

m.p. 144-145°C (acetonitrile - isopropylether)
¹H-NMR (CDCl₃): 9.58 (s, 1H, CH-triazole); 8.05 (s, 1H, CH-triazole); 7.37-6.67 (m, 3H, Ar); 6.08 (bs, H⁺); 5.80 (tt, 1H, JHF=52.6 Hz, CHF₂), AB system: VA=5.11, VB=4.87, JAB=14.2 Hz, CH₂-triazole; AB portion of an ABX system: VA=4.42, VB=4.21, JAB=11.1 Hz, JAX=7.7 Hz, JBX=2.6 Hz, CH₂O; 2.41-2.30 (m, 1H, CHCH₂); 1.32-1.18 (m, 2H, CH-CH₂); 0.83 (t, 3H, JHH=7.1 Hz, CH₃).

### (±)-1-(1H-1,2,4-triazolyl)-2-hydroxy-2-(2,4-dichlorophenyl)-3,3-dimethyl-4-(1,1,2,2-tetrafluoroethoxy)butane (Compound 8) - 67% yield

m.p. 102.5-104.5°C (hexane - isopropylether)
¹H-NMR (CDCl₃): 8.23 and 7.71 (2s, 2H, triazole); 7.86-7.12 (m, 3H, Ar); 5.57 (tt, 1H, JHF=53 Hz, CHF₂); 5.86 (s, 1H, OH); AB system: VA=5.86, VB=4.45, JAB=14.3 Hz, CH₂-triazole; AB system: VA=4.23, VB=3.80, JAB=9.8 Hz, CH₂O; 1.13 and 1.02 (2s, 6H, 2CH₃).

### (±)-1-(1H-1,2,4-triazolyl)-2-hydroxy-2-(2,4-difluorophenyl)-3,3-dimethyl-4-(1,1,2,3,3,3-hexafluoro-1-propyl)butane (Compound 9) - 18% yield

by substituting tetrafluoroethylene with 1,1,2,,3,3,3,-hexafluoropropene.
m.p. 84-85°C (hexane)
¹H-NMR (CDCl₃): 8.00 (d, 1H, CH-tetr.); 7.69 (s, 1H, CH-cat.); 7.63-6.56 (m, 3H, Ar); 5.84 (bs, 1H, OH); 5.78-4.38 (m, 2H, CH₂-triazole); 5.00-4.60 (m, 1H, CHF); 4.35-3.73 (m, 2H, OCH₂); 1.07-1.00 (m, 6H, 2CH₃).

### Example 3

### Preparation of 3-(2,4-difluorophenyl)-3,4-epoxy-2,2-dimethylbutanoic acid ethyl ester

A solution of isobutyric acid ethyl ester (1.88 g; 16 mmoles) in tetrahydrofuran (10 ml) was added dropwise to a solution of lithium diisopropylamide (prepared from n.butyllithium, 6.8 ml, 2.5 M solution in hexane and diisopropylamine, 2.38 ml, 1.72 g, 17 mmoles) in tetrahydrofuran (10 ml) at -78°C.

After 30 minutes, a solution of 1-(2,4-difluorophenyl)-2-bromoethanone (4.0 g; 17 mmoles) in tetrahydrofuran (10 ml) was added dropwise by keeping the temperature below -70°C.

The reaction mixture was kept under stirring for 90 minutes and the temperature was allowed to rise up to -30°C.

A saturated aqueous solution of ammonium chloride was added, the phases were separated and the aqueous phase was extracted with ethyl acetate (2 x 25 ml).

The collected organic phases were washed with an aqueous solution of sodium bicarbonate 5% (25 ml) and with water (25 ml), dried on sodium sulphate and evaporated under reduced pressure to give 3-(2,4-difluorophenyl)-3,4-epoxy-2,2-dimethylbutanoic acid ethyl ester (4.2 g; 96% yield) as a light yellow oil.
¹H-NMR (CDCl₃): 7.41-6.67 (m, 3H, Ar); 4.11 (q, 2H, COOCH₂); 3.26-2.71 (m, 2H, CH₂O); 1.22 (t, 3H, CH₃CH₂); 1.20 (s, 6H, 2CH₃).

### Example 4

### Preparation of 3 -(2,4-difluorophenyl)-3,4-epoxy-2,2-dimethvl-1-butanol

Lithium boron hydride (120 mg; 5.5 mmoles) was added to a solution of 3-(2,4-difluorophenyl)-3,4-epoxy-2,2-dimethylbutanoic acid ethyl ester (1g; 3.7 mmoles), prepared as described in example 3, in ethyl ether (10 ml).

After cooling at +10°C, methanol (0.22 ml; 3.5 mmoles) was added dropwise under stirring and the reaction mixture was kept at +10°C under stirring for 2 hours.

Then, a 5% hydrochloric acid solution (15 ml) was added dropwise, the phases were separated and the aqueous phase was extracted with ethyl acetate (2 x 15 ml).

The collected organic phases were washed with a 5% aqueous solution of sodium bicarbonate (20 ml) and with brine (20 ml), dried on sodium sulphate and evaporated under reduced pressure to give an oil residue (0.8 g) which was purified by column chromatography on silica gel (eluent hexane: ethyl acetate=8:2) obtaining 3-(2,4-difluorophenyl)-3,4-epoxy-2,2-dimethyl-1-butanol (823 mg; 65% yield) as a light yellow oil.
¹H-NMR (CDCl₃): 7.47-6.70 (m, 3H, Ar); 3.50-3.29 (m, 2H, CH₂OH); 3.31-2.72 (m, 2H, CH₂O); 1.00 (s, 3H, CH₃C); 0.93 (s, 3H, CH₃C).

### Example 5

### Preparation of 3-(2,4-difluorophenyl)-3,4-epoxy-2,2-dimethyl-1-butanol trifluoromethylsulphonyl ester

Trifluoromethanesulphonic anhydride (22 ml; 131 mmoles) was added dropwise to a solution of 3-(2,4-difluorophenyl)-3,4-epoxy-2,2-dimethyl-1-butanol (10 g; 44 mmoles), prepared as described in example 4, and pyridine (20.1 g; 263 mmoles) in methylene chloride (70 ml) at -20°C, while keeping the temperature below -10°C.

The reaction mixture was kept under stirring at 0°C for 30 minutes, then a 10% aqueous solution of citric acid (50 ml) was added.

The phases were separated, the aqueous phase was extracted with methylene chloride (50 ml) and the collected organic phase were washed with water (50 ml), dried on sodium sulphate and evaporated under reduced pressure at 15°C.

3-(2,4-difluorophenyl)-3,4-epoxy-2,2-dimethyl-1-butanol trifluoromethylsulphonyl ester (13 g; 81% yield) was obtained as a reddish oil which was used as such in the subsequent steps.
¹H-NMR (CDCl₃): 7.41-6.75 (m, 3H, Ar); 4.25 (s, 2H, CH₂OSO₂); 3.19-2.71 (m, 2H, CH₂O); 1.07 (s, 6H, 2CH₃C).

### Example 6

### Preparation of 2-(2,4-difluorophenyl)-2-[2-(2,2,3,3-tetrafluoropropoxy)-1,1-dimethylethyloxirane

2,2,3,3,-Tetrafluoropropanol (2.25 ml; 25 mmoles) was added dropwise to a suspension of sodium hydride (625 mg, 80%, 21 mmoles) in dimethylformamide (5 ml) at 0°C.

The mixture was kept under stirring at room temperature for 1 hour.

After cooling at -8°C, a solution of 3-(2,4-difluorophenyl)-3,4-epoxy-2,2-dimethyl-1-butanol trifluoromethylsulphonyl ester (3 g; 8.3 mmoles), prepared as described in example 5, in dimethylformamide (5 ml) was added.

The reaction mixture was kept under stirring for 6 hours at 0°C, then poured into water (200 ml) and extracted with ethyl ether (4 x 25ml).

The organic phases were dried on sodium sulphate, evaporated under reduced pressure to give an oil residue (1.58 g) which was purified by column chromatography on silica gel (eluent hexane:ethyl acetate=99:1) obtaining 2-(2,4-difluorophenyl)-2-[2-(2,2,3,3-tetrafluoropropoxy)-1,1-dimethylethyl]oxirane (1.2 g; 42% yield) as a colourless oil.
¹H-NMR (CDCl₃): 7.42-6.70 (m, 3H, Ar); 5.88 (m, 2H, OCHF); 3.72 (m, 2H, OCH₂CF₂); 3.32-3.20 (m, 2H, CH₂OCH₂CF₂); 3.12-2.64 (m, 2H, CH₂O); 0.98 and 0.97 (2s, 6H, 2CH3C).

By working in a similar way, the following compound was prepared:

### 2-(2,4-difluorophenyl)-2-[2-(2,2,2-trifluoroethoxy)-1,1-dimethylethyl]oxirane as a colourless oil

44% yield
¹H-NMR (CDCl₃): 7.42-6.70 (m, 3H, Ar); 3.82-3.69 (m, 2H, OCH₂CF₃); 3.41-3.25 (m, 2H, CH₂OCH₂CF₃); 3.19-2.64 (m, 2H, CH₂O); 0.98 and 0.97 (2s, 6H, 2CH₃C).

### Example 7

### Preparation of (±)-1-(1H-1,2,4-triazolyl)-2-hydroxy-2-(2,4-difluorophenyl)-3,3-dimethyl-4-(2,2,3,3-tetrafluoropropoxy)butane (Compound 10)

A mixture of 2-(2,4-difluorophenyl)-2-[2-(2,2,3,3-tetrafluoropropoxy)-1,1-dimethylethyl]-oxirane (1.2 g; 3.5 mmoles), 1,2,4-triazole (480 mg; 7 mmoles) and potassium carbonate (970 mg; 7 mmoles) in dimethylformamide (12 ml) was kept under stirring at 105°C for 8 hours.

The mixture was cooled, the precipitate was filtered and the solution was concentrate under reduced pressure.

The oil residue (1.5 g) was purified by column chromatography on silica gel (eluent hexane:ethyl acetate=8:2) to give compound 10 (1.1 g; 76% yield) as a white solid.
m.p. 84-86°C
¹H-NMR (CDCl₃): 8.01 (bs, 1H, triazole); 7.69 (s, 1H, triazole); 7.62-7.65 (m, 3H, Ar); 5.87 (tt, 1H, JHF=53.5 Hz, CHF₂); 5.38-4.42 (m, 2H, CH₂-triazole); 5.22 (s, 1H, OH); 3.88-3.75 (m, 2H, CH₂CF₂); AB system: VA=3.63, VB=3.28, JAB=9.3 Hz, CH₂-O; 1.01-0.96 (m, 6H, CH₃CCH₃).

By working in a similar way, the following compound was prepared:

### (±)-1-(1H-1,2,4-triazolyl)-2-hydroxy-2-(2,4-difluorophenyl)-3,3-dimethyl-4-(2,2,2-trifluoroethoxy)butane (Compound 11) - 55% yield

m.p. 71-72°C
¹H-NMR (CDCl₃): 8.01-8.00 (m, 1H, CH-triazole); 7.68 (s, 1H, CH-triazole); 7.62-6.55 (m, 3H, Ar); 5.39-4.45 (m, 2H, CH₂-triazole); 5.23 (s, 1H, OH); AB system: VA=3.84, VB=3.77, JAB=8.7 Hz, CCH₂O; AB system: VA=3.70, VB=3.30, JAB=8.8 Hz, CH₂CF₃; 1.00-0.96 (m, 6H, CH₃CCH₃).

### Example 8

### Preparation of O-[3-(2,4-difluorophenyl)-3-hydroxv-2,2-dimethyl-4-(1H-1,2,4-triazolyl)]-butyl-S-methyldithiocarbonate

A 50% aqueous solution of sodium hydroxide (25 ml) and tetrabutylammonium hydrogenosulphate (8 mg) were added to a solution of (±)-3-(2,4-difluorophenyl)-2,2-dimethyl-4-(1H-1,2,4-triazoly)-1,3-butandiol (818 mg; 2.75 mmoles) in carbon disulphide (10 ml) and methylene chloride (15 ml). Under stirring at 10°C, methyl iodide (430 mg; 3.02 mmoles) was added and the stirring was kept for 1 hour at 10°C.

The phases were separated, the aqueous phase was extracted with methylene chloride (25 ml), the collected organic phases were washed with water (25 ml), dried on sodium sulphate and evaporated to give a solid yellow residue (1.35 g) which was purified by column chromatography on silica gel (eluent ethyl acetate:hexane=7:3) obtaining O-[3-(2,4-difluorophenyl)-3-hydroxy-2,2-dimethyl-4-(1H-1,2,4-triazolyl)]butyl-S-methyldithiocarbonate (950 mg; 89% yield) as a light yellow solid.
m.p. 113-115°C
¹H-NMR (CDCl₃): 8.01 (d, 1H, CH-tetr.); 7.70 (s, 1H, CH-tetr.); 7.66-6.52 (m, 3H, Ar); 5.25-4.41 (m, 2H, CH₂-triazole); 4.71-4.40 (m, 2H, CH₂O); 2.55 (s, 3H, CH₃S); 1.11 and 1.09 (2s, 6H, 2CH₃C).

### Example 9

### Preparation of (±)-1-(1H-1,2,4-triazolyl)-2-hydroxy-2-(2,4-difluorophenyl)-3,3-dimethyl-4-trifluoromethoxy-butane (Compound 12)

The complex fluoridric acid - pyridine [(HF)₉/pyridine; 2.67 ml; 11.79 mmoles] and, then, O-[3-(2,4-difluorophenyl)-3-hydroxy-2,2-dimethyl-4-( 1H-1,2,4-triazolyl)]butyl-S-methyldithiocarbonate (520 mg; 1.34 mmoles), prepared as described in example 8, dissolved in methylene chloride (2 ml) were added dropwise to a solution of 1,3-dibromo-5,5-dimethylhydantoin (1.14 g; 4.02 mmoles) in methylene chloride (10 ml) at -70°C.

The temperature was allowed to rise to -20°C and the reaction mixture was kept under stirring for 2 hours.

The mixture was poured into a 5% aqueous solution of sodium bisulphite (25 ml) and the mixture was brought to pH 14 with 33% sodium hydroxide.

The phases were separated, the aqueous phase was extracted with methylene chloride (25 ml) and the organic phases were dried on sodium sulphate, evaporated under reduced pressure and the oil residue (480 mg) was purified by column chromatography on silica gel (eluent ethyl acetate:hexane=6:4) to give compound 12 (120 mg; 25% yield) as a white solid.
m.p. 96-98°C
¹H-NMR (CDCl₃): 8.02 (d, 1H, JHH=2.9 Hz, CH-triazole); 7.70 (s, 1H, CH-triazole); 7.63-7.57 (m, 3H, Ar); 5.37 (s, 1H, OH); AB portion of an ABX system: VA=5.26, VB=4.46, JAB=13.96 Hz, JAX=3.0 Hz, JBX=2.2 Hz, CH₂-triazole; AB system: VA=4.20, VB=3.71, JAB=9.5 Hz, CH₂OCF₃; 1.09 (d, 3H, JHF=2.4 Hz, CH₃); 0.98 (s, 3H, CH₃).

### Comparative Example A

### Preparation of (±)-1-(1H-1,2,4-triazolyl)-2-hydroxy-2-(2,4-difluorophenyl)-3,3-dimethyl-4-ethoxy-butane (Compound R1)

By working in a way similar to that described in example 6 but by using ethanol instead of 2,2,3,3-tetrafluoropropanol, the following compound was obtained.

2-(2,4-difluorophenyl)-2-(2-ethoxy-1,1-dimethyl)ethyloxirane (20% yield) as a colourless oil.
¹H-NMR (CDCl₃): 7.45-6.70 (m, 3H, Ar); 3.38 (q, 2H, CH₂CH₃); 3.20-2.60 (m, 2H, CH₂ epoxide); 3.17-3.02 (m, 2H, CH₂OCH₂CH₃); 0.98 (s, 3H, CH₃C); 0.97 (s, 3H, CH₃C).

By working in a way similar to that described in example 7, 2-(2,4-difluorophenyl)-2-(2-ethoxy-1,1-dimethyl)ethyloxirane was treated with 1,2,4-triazole obtaining compound R1 as a white solid.
35% yield
m.p. 57-58°C
¹H-NMR (CDCl₃): 8.07 (bs, 1H, CH-triazole); 7.68 (s, 1H, CH-triazole); 7.60-6.57 (m, 3H, Ar); 5.47 (bs, 1H, OH); 5.32-4.48 (m, 2H, CH₂-triazole); 3.45 (q, 2H, JHH=6.9 Hz, CH₂CH₃); AB system: VA=3.30, VB=3.22, JAB=9.5 Hz, CH₂OCH₂CH₃); 1.21 (t, 3H, CH₃CH₂); 1.02 (d, 3H, J=2.3 Hz, CH₃C); 0.94 (d, 3H, J=1.5 Hz, CH₃C).

### Comparative Example B

### Preparation of (±)-1-(1H-1,2,4-triazolyl)-2-hydroxy-2-(2,4-difluorophenyl)-3-(2,2,3,3-tetrafluoropropoxy)butane (Compound R2)

2,2,3,3-Tetrafluoropropanol (2.25 ml; 25 mmoles) was added dropwise to a suspension of sodium hydride (625 mg; 80%; 21 mmoles) in dimethylformamide (5 ml) at 0°C.

The mixture was kept under stirring at room temperature for 1 hour, then 3-(2,4-difluoro phenyl)-4-(1H-1,2,4-triazolyl)-2,3-epoxy-butane (1.25 g; 5 mmoles), prepared as described in Bull. Chem. Soc. Jap., 67 (1994), 1427-1433, dissolved in dimethylformamide (1 ml) was added and the reaction mixture was kept at 105°C for 3 hours. The mixture was poured into water (50 ml) and extracted with ethyl ether (4 x 15 ml).

The organic phases were dried on sodium sulphate, evaporated under reduced pressure to give an oil residue (1.40 g) which was purified by column chromatography on silica gel (eluent ethyl acetate:hexane=6:4) obtaining compound R2 (385 mg; 20% yield) as a white solid.
m.p. 77.0-78.5°C
¹H-NMR (CDCl₃): 7.85 and 7.72 (2s, 2H, 2CH-triazole); 7.48-6.66 (m, 3H, Ar); 5.91 (tt, 1H, JHF=54.0 Hz, CHF₂); 4.93-4.63 (m, 2H, CH₂-triazole); 4.31 (bs, 1H, OH); 4.17-3.75 (m, 3H, CHOCH₂); 1.05 (d, 3H, JHH=8.2 Hz, CH₃CH).

### Example 10

### In vitro antimycotic activity

The determination of the activity inhibiting the growth of mycetes was evaluated by the macromethod of scalar brothdilutions in geometrical progression (M.R. McGinnis and M.G. Rinaldi, "Antifungal drugs: mechanisms of action, drug resistance, susceptibility testing and assay of activity in biological fluids", in Antibiotic in Laboratory Medicine, Ed. V. Lorian, Baltimora 1991).

As culture media Yeast Nitrogen Base broth (YNB) and Sabouraud Dextrose broth (SDB) were used for yeasts and moulds, respectively.

The results obtained in SDB (after incubation at 28°C for 7 days) were expressed as minimum inhibiting concentrations (MIC) the growth of mycetes, while those obtained in YNB (after incubation at 35°C for 48 hours) were expressed as concentrations able to inhibit at 50% (IC₅₀) the growth of the yeast.

As reference compounds Fluconazole, Itraconazole and Genaconazole were used.

In the following tables the in vitro antimycotic activity data against various strains of *Candida spp*., *Aspergillus spp*., *Fusarium spp*., *Cryptococcus neoformans and Trychophyton mentagrophytes* of the some representative compounds of formula II are reported.

**Table 1**

| In vitro antimycotic activity of compounds 1-10, 12 and of the reference compound Fluconazole against *C*. *albicans*, *C*. *neoformans*, *T*. *mentagrophytes* and *A*. *fumigatus*. | | | | |
|---|---|---|---|---|
| Compound | IC₅₀ (µg/ml) | | MIC (µg/ml) | |
| | *C. albicans* 1040 | *C*. *neoformans* | *T*.*mentagrophytes* | *A*. *fumigatus* |
| 1 | 0.0156 | 0.004 | 0.125 | 8 |
| 2 | 0.0312 | 0.125 | 2 | 4 |
| 3 | 0.0625 | 0.0625 | 1 | >128 |
| 4 | 0.0312 | 0.5 | 4 | >128 |
| 5 | 0.0156 | 0.0156 | 0.25 | 8 |
| 6 | 0.0312 | 0.0625 | 0.5 | 16 |
| 7 | 0.0312 | 0.125 | 0.5 | 16 |
| 8 | 0.0312 | 0.0156 | 0.25 | >4 |
| 9 | 0.0156 | 0.125 | 2 | >128 |
| 10 | 0.0156 | 0.0625 | 0.5 | 8 |
| 12 | 0.0156 | 0.0156 | 0.125 | 4 |
| Fluconazole | 0.5 | 2 | 16 | >128 |

**Table 2**

| In vitro antimycotic activity of compounds 1, 3, 5 and of the reference compounds Fluconazole, Itraconazole and Genaconazole against *Candida spp*., *Cryptococcus neoformans* and other recently isolated strains (*Trichosporon sp*., *B*. *capitatus*, *Fusarium sp*.). | | | | | | |
|---|---|---|---|---|---|---|
| STRAINS (No.) | Compound 1 | Compound 3 | Compound 5 | Fluconazole | Itraconazole | Genaconazole |
| | MIC₅₀ MIC₉₀ | MIC₅₀ MIC₉₀ | MIC₅₀ MIC₉₀ | MIC₅₀ MIC₉₀ | MIC₅₀ MIC₉₀ | MIC₅₀ MIC₉₀ |
| *C*. *albicans* (7) | ≤0.03 0.06 | ≤0.03 2 | ≤0.03 0.5 | 4 32 | 0.06 0.12 | 2 32 |
| *C. spp* * (5) | ≤0.03 ≤0.03 | ≤0.03 ≤0.03 | ≤0.03 ≤0.03 | 1 2 | ≤0.03 0.06 | 0.5 1 |
| *C. tropicalis* (2) | 0.12 | 0.5 | 0.12 | 4-16 | 0.1 | >64 |
| *C*. *krusei* (2) | ≤0.03-0.12 | ≤0.03-0.5 | ≤0.03 0.12 | 16->64 | ≤0.03-0.12 | 16-32 |
| *C. glabrata* (2) | 0.12 | 0.5-2 | 0.12 | 32->64 | 2->16 | 32->64 |
| *C*. *neoformans* (4) | ≤0.03 ≤0.03 | ≤0.03 0.5 | ≤0.03 ≤0.03 | 1 32 | ≤0.03 0.1 | 8 16 |
| *Trichosporon sp*. (1) | ≤0.03 | 0.12 | ≤0.03 | 32 | --- | 32 |
| *B. capitatus* (1) | ≤0.03 | 0.06 | 0.06 | 4 | --- | 16 |
| *Fusarium sp*. (1) | 0,25 | 16-64 | 0.25 | --- | --- | 64 |

| | | | | | | |
|---|---|---|---|---|---|---|
| * *C. parapsilosis*, *lusitaniae*, *kefyr* | | | | | | |

**Note to the table:** MIC expressed as µg/ml
Within brackets the number of tested strains

**Table 3**

| In vitro antimycotic activity of compound 1 and of the reference compounds Itraconazole and Genaconazole against recently isolated *Aspergillus niger* and *Fusarium spp*. | | | | |
|---|---|---|---|---|
| **Species** | **Strain** | **Compound 1** | **Itraconazole** | **Genaconazole** |
| *A*. *niger* | 94-6222 | ≤0.03 | ≤0.03 | 32 |
| *Fusarium sp* | 81-256 | 0.25 | 2 | 64 |
| *Fusarium sp* | 86-3369 | 0.5 | 2-16 | --- |
| *Fusarium sp* | 88-2116 | 0.5 | 2-8 | --- |
| *Fusarium sp* | 946301 | 0.5 | 2-16 | --- |

From the data reported in the table, it results that the compounds object of the present invention have a wide-range in vitro activity. In particular, the compounds of formula II are endowed with a significant activity also against Fluconazole and Itraconazole-resistant strains (*Candida spp*. and *Cryptococcus neoformans*) and against the other pathogenic strains responsible for infections in immunodepressed patients (*C*. *glabrata*, *C*. *krusei*, *Aspergillus spp*. and *Fusarium spp*.).

### Example 11

### In vivo antimycotic activity

Charles River white mice (CD1 strain) weighing from 23 to 25 g, normo-feeded with standard diet and water *ad libitum* were used.

A suspension (0.2 ml containing 2.5x10⁷ cells/ml) of *Candida albicans* 1040 in physiologic solution was injected to each animal by venous route. Immediately after the infection and after 4, 24 and 48 hours, the animals received by oral route (in arabic gum at 2%) doses increasing in a geometrical progression of the compound to be tested. The infected group was used as control.

The observation of the mortality of the mice was prolonged up to 14 days. From the number of survived animals for each concentration the average protective dose (DP₅₀) was calculated by probits analysis (L.Lison - "Statistica applicata alla biologia sperimentale. La programmazione dell'esperimento e l'analisi dei risultati" - Casa Editrice Ambrosiana, 1961).

Compounds R1 and R2 were used as comparative compounds.

Fluconazole and Itraconazole were used as reference compounds.

The in vivo antimycotic activity data of some representative compounds of formula II after in vivo administration are reported in the following tables:

**Table 4**

| In vivo antimycotic activity of compounds 1-3 and of the reference compound Itraconazole in mice experimentally infected by *C*. *albicans*, expressed as percentage of survival. | | | |
|---|---|---|---|
| **Compound** | **dose mg/kg/os** | **% percentage of survival after day** | |
| | | **3** | **6** |
| 1 | 25 | 100 | 100 |
| 2 | 25 | 100 | 100 |
| 3 | 25 | 100 | 100 |
| Itraconazole | 25 | 100 | 100 |
| Control | --- | 50 | 0 |

**Table 5**

| Antimycotic efficacy by oral route of compounds 1, 3-7, 9, 12, R1, R2 and of the reference compounds Fluconazole and Itraconazole, expressed as average protective dose (PD₅₀) after 8 and 14 days from the infection. | | |
|---|---|---|
| **Compound** | **PD**_{**50**} **(mg/kg/os) at 8th day** | **PD**_{**50**} **(mg/kg/os) at 14th day** |
| 1 | 0.120 | 0.74 |
| 3 | 0.49 | 1.3 |
| 4 | 0.31 | 1.29 |
| 5 | 0.50 | 1.14 |
| 6 | 0.42 | 1.10 |
| 7 | 1.5 | 2.19 |
| 9 | 0.89 | 1.57 |
| 12 | 1.09 | 1.39 |
| R₁ | >4 | >4 |
| R₂ | >4 | >4 |
| Itraconazole | 2.31 | >8 |
| Fluconazole | 0.125 | 0.61 |

From the reported data it results that the compounds of formula II, object of the present invention, are active by oral administration at an average protective dose lower than that of Itraconazole and at least equivalent to that of Fluconazole.

Furthermore, the data reported in table 5 suggest, particularly for compound 1, characteristics similar to those of Fluconazole.

## Claims

1. A compound of formula 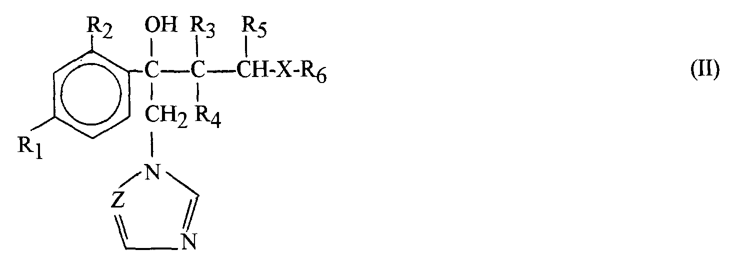 wherein
R₁ is chlorine, fluorine or trifluoromethyl;
R₂ is hydrogen, chlorine, fluorine or trifluoromethyl;
Z is CH or N;
R₃, R₄ and R₅, the same or different, are hydrogen or C₁-C₄ alkyl, provided that when one of R₃ or R₄ is hydrogen, the other of R₃ or R₄ is different from R₅;
X is O or S;
R₆ is a C₁-C₅ polyfluoroalkyl group containing at least two fluorine atoms and optionally other halogen atoms selected among chlorine and bromine;
and their salts with pharmaceutically acceptable acids;
for use as a medicament.

2. A compound according to claim 1 wherein R₃, R₄ and R₅, the same or different, are hydrogen, methyl or ethyl.

3. A compound according to claim 1 or 2 wherein R₆ is selected among difluoromethyl, trifluoromethyl, 1,1,2-trifluoro-2-chloroethyl, 1,1-difluoroethyl, 2,2,2-trifluoroethyl, 1,1,2-trifluoroethyl, 1,1,2,3,3,3-hexafluoropropyl, 2,2,3,3-tetrafluoropropyl, 1,1,2,2-tetrafluoroethyl groups and their isomers.

4. A compound according to claim 3 wherein R₆ is a 1,1,2,2-tetrafluoroethyl group.

5. A compound according to claim 1 wherein R₁ is chlorine or fluorine, R₂ is hydrogen, chlorine or fluorine, R₃ is methyl, R₄ and R₅, the same or different, are hydrogen, methyl or ethyl, Z is N and R₆ is a 1,1,2,2-tetrafluoroethyl group.

6. A compound according to claim 1 wherein R₁ is chlorine or fluorine, R₂ is hydrogen, chlorine or fluorine, R₃ is methyl, R₄ and R₅, the same or different, are hydrogen, methyl or ethyl, Z is N and R₆ is a 1,1,2,2-tetrafluoroethyl group and X is O.

## Patentansprüche

1. Verbindung der Formel 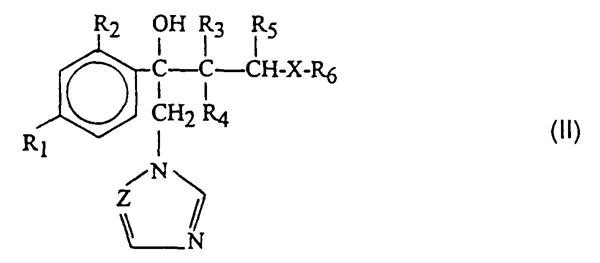 wobei
R₁ Chlor, Fluor oder Trifluormethyl ist;
R₂ Wasserstoff, Chlor, Fluor oder Trifluormethyl ist;
Z gleich CH oder N ist;
R₃, R₄ und R₅ gleich oder voneinander verschieden Wasserstoff oder C₁-C₄-Alkyl sind, unter der Voraussetzung, daß dann, wenn eine der Gruppen R₃ oder R₄ Wasserstoff ist, die andere der Gruppen R₃ oder R₄ von R₅ verschieden ist;
X gleich O oder S ist; und
R₆ eine C₁-C₅-Polyfluoralkylgruppe ist, die mindestens zwei Fluoratome und wahlweise andere aus Chlor und Brom ausgewählte Halogenatome enthält; sowie ihre Salze mit pharmazeutisch akzeptablen Säuren;
zur Verwendung als Arzneimittel.

2. Verbindung gemäß Anspruch 1, wobei R₃, R₄ und R₅ gleich oder voneinander verschieden Wasserstoff, Methyl oder Ethyl sind.

3. Verbindung gemäß Anspruch 1 oder 2, wobei R₆ aus Difluormethyl-, Trifluormethyl-, 1,1,2-Trifluor-2-chlorethyl-, 1,1-Difluorethyl-, 2,2,2-Trifluorethyl-, 1,1,2-Trifluorethyl-, 1,1,2,3,3,3-Hexafluorpropyl-, 2,2,3,3-Tetrafluorpropyl- und 1,1,2,2-Tetrafluorethylgruppen sowie deren Isomeren ausgewählt ist.

4. Verbindung gemäß Anspruch 3, wobei R₆ eine 1,1,2,2-Tetrafluorethylgruppe ist.

5. Verbindung gemäß Anspruch 1, wobei R₁ Chlor oder Fluor, R₂ Wasserstoff, Chlor oder Fluor, R₃ Methyl, R₄ und R₅ gleich oder voneinander verschieden Wasserstoff, Methyl oder Ethyl, Z gleich N und R₆ eine 1,1,2,2-Tetrafluorethylgruppe ist.

6. Verbindung gemäß Anspruch 1, wobei R₁ Chlor oder Fluor, R₂ Wasserstoff, Chlor oder Fluor, R₃ Methyl, R₄ und R₅ gleich oder voneinander verschieden Wasserstoff, Methyl oder Ethyl, Z gleich N, R₆ eine 1,1,2,2-Tetrafluorethylgruppe und X gleich O ist.

## Revendications

1. Composé de formule 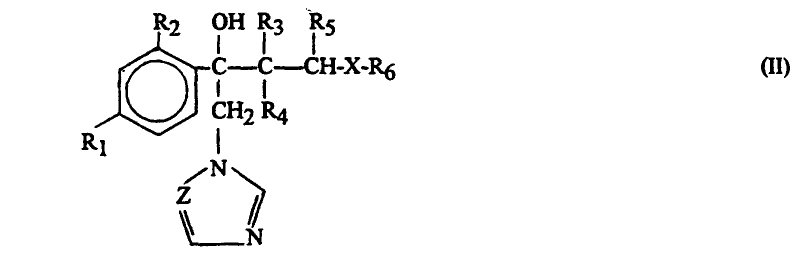 dans laquelle
R₁ est du chlore, du fluor ou un groupe trifluorométhyle ;
R₂ est de l'hydrogène, du chlore, du fluor ou un groupe trifluorométhyle ;
Z est CH ou N ;
R₃, R₄ et R₅, identiques ou différents, sont de l'hydrogène ou un groupe alkyle en C₁ à C₄, étant entendu que lorsque l'un parmi R₃ et R₄ est de l'hydrogène, l'autre parmi R₃ et R₄ est différent de R₅ ;
X est O ou S ;
R₆ est un groupe polyfluoroalkyle en C₁ à C₅ contenant au moins deux atomes de fluor et éventuellement d'autres atomes d'halogène choisis parmi le chlore et le brome ;
et leurs sels avec des acides pharmaceutiquement acceptables ;
pour l'utilisation en tant que médicament.

2. Composé selon la revendication 1, dans lequel R₃, R₄ et R₅, identiques ou différents, sont de l'hydrogène, un groupe méthyle ou éthyle.

3. Composé selon la revendication 1 ou 2, dans lequel R₆ est choisi parmi les groupes difluorométhyle, trifluorométhyle, 1,1,2-trifluoro-2-chloroéthyle, 1,1-difluoroéthyle, 2,2,2-trifluoroéthyle, 1,1,2-trifluoroéthyle, 1,1,2,3,3,3-hexafluoropropyle, 2,2,3,3-tétrafluoropropyle, 1,1,2,2-tétrafluoroéthyle et leurs isomères.

4. Composé selon la revendication 3, dans lequel R₆ est un groupe 1,1,2,2-tétrafluoroéthyle.

5. Composé selon la revendication 1, dans lequel R₁ est du chlore ou du fluor, R₂ est de l'hydrogène, du chlore ou du fluor, R₃ est un groupe méthyle, R₄ et R₅, identiques ou différents, sont de l'hydrogène, un groupe méthyle ou éthyle, Z est N et R₆ est un groupe 1,1,2,2-tétrafluoroéthyle.

6. Composé selon la revendication 1, dans lequel R₁ est du chlore ou du fluor, R₂ est de l'hydrogène, du chlore ou du fluor, R₃ est un groupe méthyle, R₄ et R₅, identiques ou différents, sont de l'hydrogène, un groupe méthyle ou éthyle, Z est N et R₆ est un groupe 1,1,2,2-tétrafluoroéthyle et X est O.
